(19) Europäisches Patentamt
European Patent Office
Office européen des brevets

(11) **EP 4 708 154 A1**

(12) **EUROPEAN PATENT APPLICATION**

(43) Date of publication:
**11.03.2026 Bulletin 2026/11**

(21) Application number: **25200782.8**

(22) Date of filing: **08.09.2025**

(51) International Patent Classification (IPC):
**G06N 5/022** (2023.01)    **G06N 3/0475** (2023.01)

(52) Cooperative Patent Classification (CPC):
**G06N 5/022; G06N 3/0475; G16H 50/20; G16H 50/70; G16H 80/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **06.09.2024 CN 202411252149**

(71) Applicant: **Alipay (Hangzhou) Information Technology Co., Ltd.**
**Hangzhou, Zhejiang 310000 (CN)**

(72) Inventors:
• **ZHONG, Jialun**
  **Hangzhou, Zhejiang, 310000 (CN)**

• **LI, Yanzeng**
  **Hangzhou, Zhejiang, 310000 (CN)**
• **HU, Sen**
  **Hangzhou, Zhejiang, 310000 (CN)**
• **ZHANG, Yang**
  **Hangzhou, Zhejiang, 310000 (CN)**
• **CHEN, Yicheng**
  **Hangzhou, Zhejiang, 310000 (CN)**
• **XU, Teng**
  **Hangzhou, Zhejiang, 310000 (CN)**

(74) Representative: **Fish & Richardson P.C.**
**Highlight Business Towers**
**Mies-van-der-Rohe-Straße 8**
**80807 München (DE)**

(54) **MEDICAL LLM MODEL INFERENCE METHOD BASED ON KNOWLEDGE GRAPH AND RELATED DEVICES**

(57)    This specification provides a medical LLM model inference method based on a knowledge graph and related devices. The knowledge graph includes a plurality of nodes and edges connecting the nodes, the nodes represent entities, and the edges represent relationships between the entities. The method includes: obtaining target dialogue text entered by a user, and identifying, from the knowledge graph, a subgraph related to an entity mention included in the target dialogue text, where the subgraph includes a target node that represents a target entity and a plurality of neighboring nodes connected to the target node; obtaining attribute values of a plurality of attributes of the target entity from multi-turn dialogue text that is entered by the user and that includes the target dialogue text; constructing, based on the subgraph and the attribute values of the plurality of attributes of the target entity, a dialogue graph corresponding to the target dialogue text; and constructing a prompt based on the dialogue graph and the target dialogue text, and inputting the prompt into an LLM model, so that the LLM model performs logical inference based on the prompt.

| |
|---|
| Obtain target dialogue text entered by a user, and identify, from a knowledge graph, a subgraph related to an entity mention included in the target dialogue text, where the subgraph includes a target node that represents a target entity and a plurality of neighboring nodes connected to the target node — S301 |
| Obtain attribute values of a plurality of attributes of the target entity from multi-turn dialogue text that is entered by the user and that includes the target dialogue text — S302 |
| Construct, based on the subgraph and the attribute values of the plurality of attributes of the target entity, a dialogue graph corresponding to the target dialogue text — S303 |
| Construct a prompt based on the dialogue graph and the target dialogue text, and input the prompt into an LLM model, so that the LLM model performs logical inference based on the prompt — S304 |

FIG. 3

EP 4 708 154 A1

Description

TECHNICAL FIELD

One or more embodiments of this specification relate to the field of large model technologies, and in particular, to a medical Large Language Model (LLM)I inference method based on a knowledge graph and related devices.

BACKGROUND

A large language model (LLM), or referred to as a pre-training model, is a language model constructed by using a deep neural network that includes more than tens of billions of parameters, that has been pre-trained using a self-supervised learning method on large and diversified public datasets to acquire many language phenomena.
Further, to apply the LLM model to a specific application scenario, further fine-tuning training can be performed on a pre-training completed LLM model by using a dataset related to the specific application scenario, and the fine-tuned LLM model can be used to execute a multi-turn dialogue task related to the specific application scenario.
However, the fine-tuned LLM model still has many defects when executing the multi-turn dialogue task. For example, there is still a lack of specialized knowledge related to the application scenario, especially long tail knowledge, and it is difficult to remember all detailed information included in a multi-turn dialogue history. Finally, a hallucination may be caused in dialogue content generated by the LLM model, in other words, content is unreliable and inaccurate, and cannot satisfy an actual use need of a user, and even incorrect inference provided by the LLM model may cause serious harm to the user.

SUMMARY

In view of this, one or more embodiments of this specification provide a medical LLM model inference method based on a knowledge graph and related devices.
According to a first aspect, this specification provides an LLM model inference method based on a knowledge graph, where the knowledge graph includes a plurality of nodes and edges connecting the nodes, the nodes represent entities, and the edges represent relationships between the entities; and the method includes: obtaining target dialogue text entered by a user, and identifying, from the knowledge graph, a subgraph related to an entity mention included in the target dialogue text, where the subgraph includes a target node that represents a target entity and a plurality of neighboring nodes connected to the target node; obtaining attribute values of a plurality of attributes of the target entity from multi-turn dialogue text that is entered by the user and that includes the target dialogue text; constructing, based on the subgraph and the attribute values of the plurality of attributes of the target entity, a dialogue graph corresponding to the target dialogue text; and constructing a prompt based on the dialogue graph and the target dialogue text, and inputting the prompt into an LLM model, so that the LLM model performs logical inference based on the prompt.
According to a second aspect, this specification provides an LLM model inference apparatus based on a knowledge graph, where the knowledge graph includes a plurality of nodes and edges connecting the nodes, the nodes represent entities, and the edges represent relationships between the entities; and the apparatus includes: a first acquisition unit, configured to obtain target dialogue text entered by a user, and identify, from the knowledge graph, a subgraph related to an entity mention included in the target dialogue text, where the subgraph includes a target node that represents a target entity and a plurality of neighboring nodes connected to the target node; a second acquisition unit, configured to obtain attribute values of a plurality of attributes of the target entity from multi-turn dialogue text that is entered by the user and that includes the target dialogue text; a dialogue graph construction unit, configured to construct, based on the subgraph and the attribute values of the plurality of attributes of the target entity, a dialogue graph corresponding to the target dialogue text; and an inference unit, configured to construct a prompt based on the dialogue graph and the target dialogue text, and input the prompt into an LLM model, so that the LLM model performs logical inference based on the prompt.
Correspondingly, this specification further provides a computing device, including a memory and a processor, where the memory stores a computer program/instructions that can be run by the processor; and when running the computer program/instructions, the processor executes the LLM model inference method based on a knowledge graph according to the above-mentioned first aspect.
Correspondingly, this specification further provides a computer-readable storage medium, where a computer program/instructions is/are stored on the computer-readable storage medium; and when being run by a processor, the computer program/instructions executes/execute the LLM model inference method based on a knowledge graph according to the above-mentioned first aspect.
Correspondingly, this specification further provides a computer program product, where the computer program product includes a computer program/instructions, and when being executed by a processor, the computer program/instructions executes/execute the LLM model inference method based on a knowledge graph according to the above-mentioned first aspect.

In conclusion, in this application, after the target dialogue text entered by the user is obtained, on the one hand, the subgraph related to the target entity corresponding to the entity mention included in the target dialogue text can be searched from the knowledge graph, to assist in enhancing specialized knowledge of the LLM model. On the other hand, it is considered that the plurality of attributes of the entity (for example, a symptom and duration) are critical to inference of the LLM model. Therefore, the attribute values of the plurality of attributes of the entity can be obtained from the multi-turn dialogue text of the user to capture fine-grained information in a dialogue of the user. Then, a clear and detailed dialogue graph is constructed based on the obtained subgraph and the attribute values of the plurality of attributes of the entity. This dialogue graph, which encapsulates both specialized knowledge and fine-grained user conversation information, then aids the LLM model in its logical inference. This approach significantly improves the LLM model's inference performance and ensures the accuracy of its reasoning results.

## BRIEF DESCRIPTION OF DRAWINGS

FIG. 1 is a diagram illustrating a medical dialogue, according to some example embodiments.
FIG. 2 is a diagram illustrating a system architecture, according to some example embodiments.
FIG. 3 is a schematic flowchart illustrating an LLM model inference method based on a knowledge graph, according to some example embodiments.
FIG. 4 is a schematic flowchart illustrating construction of a dialogue graph, according to some example embodiments.
FIG. 5 is a diagram illustrating generation of prompt text based on a dialogue graph, according to some example embodiments.
FIG. 6 is another diagram illustrating generation of prompt text based on a dialogue graph, according to some example embodiments.
FIG. 7 is still another diagram illustrating generation of prompt text based on a dialogue graph, according to some example embodiments.
FIG. 8 is a diagram of a structure of an LLM model inference apparatus based on a knowledge graph, according to some example embodiments.
FIG. 9 is a diagram illustrating a structure of a computing device, according to some example embodiments.

## DESCRIPTION OF EMBODIMENTS

Example embodiments are described in detail here, and examples of the example embodiments are represented in the accompanying drawings. When the following description relates to the accompanying drawings, unless specified otherwise, same numbers in different accompanying drawings represent same or similar elements. The implementations described in the following example embodiments do not represent all implementations consistent with one or more embodiments of this specification. On the contrary, the implementations are only examples of apparatuses and methods that are described in the appended claims in detail and consistent with some aspects of one or more embodiments of this specification.

It is worthwhile to note that, steps of corresponding methods in other embodiments are not necessarily performed in the order shown and described in this specification. In some other embodiments, the method can include more or less steps than those described in this specification. In addition, a single step described in this specification may be divided into a plurality of steps for description in other embodiments, and a plurality of steps described in this specification may also be combined into a single step for description in other embodiments.

It is worthwhile to note that "a plurality of" mentioned in this application means two or more.

In addition, user information (including but not limited to user equipment information and user personal information) and data (including but not limited to data used for analysis, stored data, and displayed data) involved in this application are information and data that are authorized by a user or that are fully authorized by each party, and related data needs to be collected, used, and processed in compliance with relevant national and regional laws, regulations, and standards, and be provided with a corresponding operation portal for the user to authorize or reject.

As described above, after a pre-training completed LLM model is fine-tuned by using a dataset related to a specific application scenario, the fine-tuned LLM model can execute a multi-turn dialogue task related to the specific application scenario, but there are still many defects when the fine-tuned LLM model executes the multi-turn dialogue task, which cannot satisfy an actual use need of a user.

For example, the specific application scenario is a medical consultation scenario. A fine-tuned LLM model can be a medical LLM model. A complete medical dialogue system (MDS) can be further constructed based on the medical LLM model, to implement human-machine interactive consultation in a medical field, including analyzing input of a patient, and making corresponding disease diagnosis, treatment advice, medicine recommendation, etc. Details are not described here again.

A plurality of turns of dialogues between the user and the medical LLM model can be denoted as $H = (p_1, d_1, ..., p_{N-1}, d_{N-1}, p_N)$, where $P = (p_1, ..., p_N)$ is a plurality of turns of dialogues entered by the user, and $D = (d_1, ..., d_{N-1})$ is a plurality of turns of dialogues output by the medical LLM model. A final generation target of the medical LLM model can be to generate a dialogue $d_N$. The dialogue $d_N$ can include a final disease diagnosis result, treatment advice, medicine recommendation, etc.

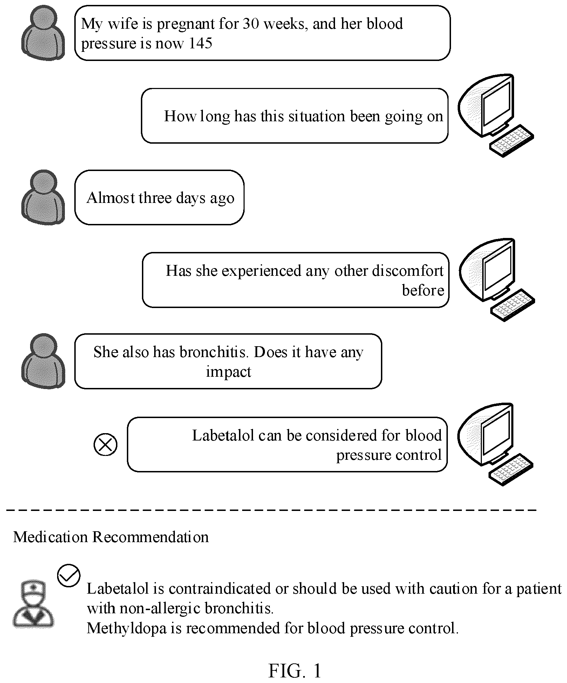

However, there are still many defects in a current medical LLM model. Referring to FIG. 1, FIG. 1 is a diagram illustrating a medical dialogue, according to some example embodiments. As shown in FIG. 1, a plurality of turns of dialogues can be performed between a patient and a medical LLM model. Finally, the medical LLM model needs to provide recommended medicine for the patient. According to content shown in FIG. 1, it can be obtained through analysis that the following disadvantages still exist in a current medical LLM model:

(1) Coreference resolution: It is difficult to determine whether the medical LLM model can understand a medical meaning specifically represented by "this case" in the figure, for example, "blood pressure 145" represents "hypertension", in other words, the medical LLM model may not connect a specific symptom described by the patient to a professional medical meaning, affecting subsequent diagnosis.

(2) Forgetfulness of fine-grained information of a multi-turn dialogue history: After the plurality of turns of dialogues are performed, it is difficult for the medical LLM model to remember fine-grained medical information included in the dialogue history.

(3) Lack of specialized knowledge: Although the medical LLM model performs fine-tuning training on a large amount of medical data, it is usually difficult to remember complex specialized knowledge in the medical field, especially long tail knowledge. As shown in FIG. 1, due to a lack of specialized knowledge "Patients with non-allergic bronchitis should avoid or use Labetalol with caution." in the medical LLM model, only knowledge that commonly used medicine of hypertension is Rabbellol is acquired. Consequently, incorrect medication recommendation "Rabbellol can be considered to control the blood pressure" is directly exported to the patient, which seriously endangers the health of the patient.

(4) Hallucination phenomenon: The above shortcomings can all lead to hallucination in generated content of the medical LLM model, in other words, outputting unreasonable, unreliable, and inaccurate consultation results (including an incorrect disease diagnosis result, incorrect treatment advice, and incorrect medicine recommendation) to the patient, which seriously endangers the health of the patient.

Based on this, this specification provides a technical solution. A dialogue graph is constructed, to explicitly capture fine-grained information included in a plurality of turns of dialogues, preventing forgetfulness of an LLM model, and enhance specialized knowledge by using a knowledge graph, avoiding hallucination generated by the LLM model, thereby further comprehensively improving an inference capability of the LLM model and ensuring reliability and correctness of an inference result.

During implementation, target dialogue text entered by a user can be first obtained, and a subgraph related to a target entity corresponding to an entity mention included in the target dialogue text is found from the knowledge graph. The subgraph can include a target node that represents the target entity and a plurality of neighboring nodes connected to the target node. Further, attribute values of a plurality of attributes of the target entity can be obtained from multi-turn dialogue text that is entered by the user and that includes the target dialogue text. Further, a dialogue graph corresponding to the target dialogue text is constructed based on the obtained subgraph and the attribute values of the plurality of attributes of the target entity. Further, a corresponding prompt can be constructed based on the dialogue graph and the target dialogue text, and the prompt is inputted into the LLM model, so that the LLM model performs logical inference based on the prompt.

In the above technical solution, after the target dialogue text entered by the user is obtained, on the one hand, the subgraph related to the target entity corresponding to the entity mention included in the target dialogue text is searched from the knowledge graph, to assist in enhancing specialized knowledge of the LLM model. On the other hand, it is considered that the plurality of attributes of the entity (for example, a symptom and duration) are critical to inference of the LLM model. Therefore, the attribute values of the plurality of attributes of the entity can be obtained from the multi-turn dialogue text of the user to capture fine-grained information in a dialogue of the user. Then, a clear and detailed dialogue graph is constructed based on the obtained subgraph and the attribute values of the plurality of attributes of the entity. This dialogue graph, which encapsulates both specialized knowledge and fine-grained user conversation information, then aids the LLM model in its logical inference. This approach significantly improves the LLM model's inference performance and ensures the accuracy of its reasoning results. Especially in the medical field, the reliability of the LLM model for performing a medical dialogue can be improved, thereby avoiding endangerment to the health of the user caused by incorrect diagnosis of the LLM model.

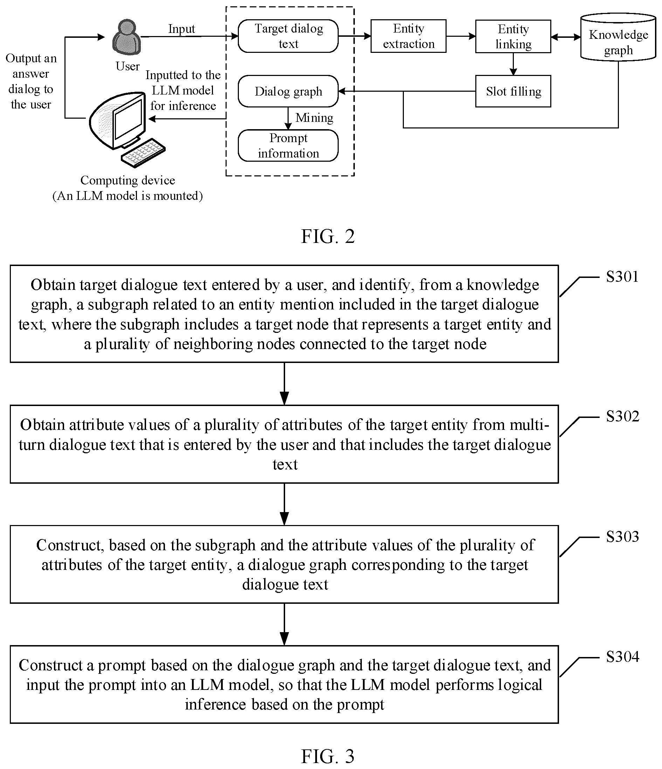

Referring to FIG. 2, FIG. 2 is a diagram illustrating a system architecture, according to some example embodiments. As shown in FIG. 2, one or more embodiments provided in this specification can be specifically implemented in the system architecture shown in FIG. 2 or a similar system architecture.

As shown in FIG. 2, an LLM model is mounted on a computing device. The LLM model can be an LLM service model obtained after a pre-trained LLM base model is fine-tuned based on data related to a target application scenario, and can be used to execute a multi-turn dialogue task related to the target application scenario. In a shown implementation, the user can perform a plurality of turns of dialogues with the LLM model by using an input device (such as a keyboard or a touchscreen) provided by the computing device, to perform consultation related to the target application scenario on the LLM model and obtain a corresponding answer.

In a shown implementation, the above-mentioned target application scenario can be a medical consultation scenario, a life assistant scenario, an emotion analysis scenario, etc. This is not specifically limited in this specification.

As shown in FIG. 2, the user can enter the target dialogue text through the computing device. Correspondingly, the computing device can obtain the target dialogue text entered by the user. For example, an example in which the target application scenario is a medical consultation scenario is used. The target dialogue text can be "My wife is pregnant for 30 weeks, and her blood pressure is now 145" shown in FIG. 1, "Almost three days ago", "She also has bronchitis. Does it have any impact". This is not specifically limited in this specification.

Further, as shown in FIG. 2, the computing device can perform entity extraction on the obtained target dialogue text to extract an entity mention (mention) included in the target dialogue text. For details, refer to the following descriptions of the embodiment corresponding to FIG. 3. Details are not described here again. For example, an example in which "My wife is pregnant for 30 weeks, and her blood pressure is now 145" shown in FIG. 1 in the above is the target dialogue text is used. The extracted entity mention can be "pregnant".

Further, as shown in FIG. 2, it is considered that the entity mention included in the target dialogue text entered by the user may refer to an irregular case such as an abbreviation, an omission, and an alias. It is necessary to map (or link) the entity mention to a unified and standard entity in the knowledge graph. The knowledge graph usually includes a plurality of nodes and edges connecting the nodes, the nodes represent entities, and the edges represent relationships between the entities.

In a shown implementation, the target entity that is included in the knowledge graph and that corresponds to the entity mention can be determined in an entity linking (EL) way. Further, the subgraph related to the target entity can be further found from the knowledge graph. For details, refer to the following descriptions of the embodiment corresponding to FIG. 3. Details are not described here again. For example, an example in which "My wife is pregnant for 30 weeks, and her blood pressure is now 145" shown in FIG. 1 in the above is the target dialogue text is still used, where an included entity mention is "pregnant". In this case, a target entity that is entity linked to in the knowledge graph of the medical field may also be "pregnant" or may be "pregnancy". This is not specifically limited in this specification.

Further, as shown in FIG. 2, it is considered that attribute values of attributes of the entity, for example, duration of pregnancy, duration of symptom generation, a specific value of blood pressure, and a specific part of pain, that are used as inference conditions greatly affect an inference result of the LLM model. Therefore, detailed information related to the target entity can be further obtained from multi-turn dialogue text that is entered by the user and that includes the target dialogue text. For details, refer to the following descriptions of the embodiment corresponding to FIG. 3. Details are not described here again.

Further, as shown in FIG. 2, a corresponding dialogue graph can be obtained through construction based on the subgraph that is included in the knowledge graph and that is related to the target entity and the detailed information that is obtained from the multi-turn dialogue text entered by the user and that is related to the target entity. For details, refer to the following descriptions of the embodiment corresponding to FIG. 3. Details are not described here again.

Further, as shown in FIG. 2, after the dialogue graph is obtained through construction, key prompt information can be further obtained by mining the dialogue graph. Finally, the target dialogue text, the dialogue graph, and the prompt information can be inputted together into the LLM model for inference to obtain a corresponding inference result and output the result to the user, in other words, an answer dialogue corresponding to the target dialogue text is output, and so on.

Therefore, in this application, by constructing the dialogue graph, on the one hand, fine-grained information included in the plurality of turns of dialogues is explicitly captured, to avoid forgetfulness of the LLM model, and on the other hand, specialized knowledge is enhanced by using the knowledge graph, to avoid hallucination generated by the LLM model, thereby further comprehensively improving an inference capability of the LLM model and ensuring reliability and correctness of the inference result.

In a shown implementation, the computing device on which the LLM model is mounted can be, for example, an intelligent wearable device, a smartphone, a tablet computer, a notebook computer, a desktop computer, etc. This is not specifically limited in this specification.

Referring to FIG. 3, FIG. 3 is a schematic flowchart illustrating an LLM model inference method based on a knowledge graph, according to some example embodiments. The method can be applied to the computing device shown in FIG. 2. As shown in FIG. 3, the method can specifically include step S301 to step S304 in the following.

Step S301: Obtain target dialogue text entered by a user, and identify, from the knowledge graph, a subgraph related to an entity mention included in the target dialogue text, where the subgraph includes a target node that represents a target entity and a plurality of neighboring nodes connected to the target node.

First, the computing device can obtain the target dialogue text entered by the user, where the target dialogue text can be any turn of dialogue text entered by the user in a process of performing a multi-turn dialogue with an LLM model. Then, the computing device can perform entity extraction on the target dialogue text, to extract the entity mention included in the target dialogue text.

It is worthwhile to note that a specific implementation of the entity extraction is not particularly limited in this specification.

In a shown implementation, the target dialogue text and a predetermined entity extraction instruction can be inputted into the LLM model, so that the LLM model extracts, based on the entity extraction instruction, the included entity mention from the target dialogue text. In a shown implementation, the LLM model can perform instruction fine-tuning on an information extraction dataset in advance. This is not specifically limited in this specification.

For example, a formula corresponding to the entity extraction can be: C = LLM ($I_{NER}$, $p_m$), where C is an entity mention set constructed by a plurality of extracted entity mentions, $I_{NER}$ is an entity extraction instruction, and $p_m$ is an $m^{th}$ turn of dialogue text (namely, the target dialogue text) entered by the user.

It is worthwhile to note that specific content of the entity extraction instruction $I_{NER}$ is not particularly limited in this specification.

In a shown implementation, the entity extraction instruction $I_{NER}$ can include an extraction prompt and a plurality of examples. For example, the entity extraction instruction $I_{NER}$ can be shown as follows:

You are a named entity identification annotator in the medical field. Given a section of medical dialogue text, disease entities need to be returned in a form of a list. A reference format is as follows: ["Entity 1", "Entity 2", ...]

Input example 1:

Patient: I just got tested at the hospital, and the result shows HBsAg positive (+). What does this mean, and what medicine should I take to recover?

Output Example 1: ["Hepatitis B"]

Input example 2:

Patient: I have a cold but no fever, with sneezing, watery nose, and swollen eyes.
Doctor: Hello. How long has this situation been going on?
Patient: I've been coughing for three days.
Doctor: Is there any sputum when you cough? White or yellow sputum?
Patient: White sputum.
Patient: I sweat a lot.
Doctor: I see. What medicine have you taken these days?
Patient: I haven't taken any medicine.
Doctor: You have a viral cold.
Patient: Do I need an intravenous drip?
Doctor: I recommend taking [MASK], [MASK], and [MASK].

Output Example 2: ["Cold", "Viral Cold"]

Input example 3:

Patient: The pharmacist said it's probably chronic urticaria. It's been over half a year. I often feel itchy around my lower abdomen where the belt presses, and scratching makes it worse, leaving red patches. Other areas occasionally itch too. I also have allergic rhinitis, and my eyes are also very sensitive. I think they might be related. Taking loratadine helps now. In addition, the pharmacist also recommended I take BaiXuanXiaTaRePian, vitamin E, and calcium supplements for a few months, claiming it could cure it completely?

Doctor: Hello. The treatment of the chronic urticaria needs continuous medication for four weeks, followed by gradual dose reduction over another four weeks. Oral loratadine is sufficient. All other medicines are just supplementary.

Output Example 3: ["Chronic urticaria", "Allergic rhinitis"]

Further, after the entity mention is extracted from the target dialogue text entered by the user, the entity mention in the target dialogue text can be correctly linked to an entity in the knowledge graph (or a knowledge base) without ambiguity in an entity linking way, in other words, the target entity that is included in the knowledge graph and that corresponds to the entity mention is determined.

It is worthwhile to note that a specific implementation of entity linking is not particularly limited in this specification.

In a shown implementation, a synonym word table in the knowledge graph can be used for mapping, to map the entity mention extracted from the target dialogue text to a target entity that is included in the knowledge graph and that is a synonym of the entity mention. Alternatively, synonym matching can be performed, based on the synonym word table, on the entity mention extracted from the target dialogue text and entities represented by the plurality of nodes included in the knowledge graph. If the entity mention and a target entity represented by a target node in the knowledge graph are synonyms that match each other, the entity mention can be linked to the target entity that is in the knowledge graph and that corresponds to the entity mention.

In a shown implementation, the entity linking can alternatively be converted into a classification task oriented to a knowledge graph entity, and processing is performed by using a mapping classification model on which training is already completed, to determine a target entity that is included in the knowledge graph and that corresponds to the entity mention.

In a shown implementation, matching can alternatively be performed, by using a heuristic method such as a longest substring, on the entity mention and entities represented by the plurality of nodes included in the knowledge graph, to determine a target entity that is included in the knowledge graph and that corresponds to the entity mention, etc. This is not specifically limited in this specification.

For example, the entity mention extracted from the target dialogue text can be "increased nocturia", and a target entity that is in the knowledge graph and that corresponds to the entity mention can be "frequent micturition". For example, the entity mention extracted from the target dialogue text can be "pregnant", and a target entity that is in the knowledge graph and that corresponds to the entity mention can be "pregnant", "pregnancy", etc. This is not specifically limited in this specification.

Further, after the target entity that is included in the knowledge graph and that corresponds to the entity mention is determined, a subgraph related to the target entity can be further found from the knowledge graph. The subgraph can include a target node that represents the target entity and a plurality of neighboring nodes connected to the target node.

Step S302: Obtain attribute values of a plurality of attributes of the target entity from multi-turn dialogue text that is entered by the user and that includes the target dialogue text.

Further, it is considered that states of the entity, to be specific, attribute values of attributes of the entity, such as duration of pregnancy, duration of symptom generation, a specific value of blood pressure, and a specific part of pain, that are used as inference conditions greatly affect an inference result of the LLM model, and the user usually does not express all detailed information of the user in one turn of dialogue. Therefore, in this application, detailed information related to the target entity (namely, the attribute values of the plurality of attributes of the target entity) can be further obtained from the multi-turn dialogue text that is entered by the user and that includes the target dialogue text.

It is worthwhile to note that a specific implementation of obtaining the attribute values of the plurality of attributes of the target entity is not particularly limited in this specification.

In a shown implementation, the attribute values of the plurality of attributes of the target entity can be obtained in a slot filling way.

Specifically, the plurality of attributes of the target entity can be first determined based on an entity type of the target entity, and then a plurality of predetermined slots corresponding to the plurality of attributes are predefined. Then, slot values of the plurality of predetermined slots corresponding to the target entity are obtained, in the slot filling way, from the multi-turn dialogue text that is entered by the user and that includes the target dialogue text.

For example, assume that the target entity is "waist pain". Correspondingly, the entity type of the target entity is a symptom. In this case, the plurality of attributes (slots) of the target entity can include duration, location, etc. Correspondingly, the plurality of attribute values (slots) can be respectively three days and a left side. This is not specifically limited in this specification.

In a shown implementation, when the slot values of the plurality of predetermined slots corresponding to the target entity are obtained, in the slot filling manner, from the multi-turn dialogue text that is entered by the user and that includes the target dialogue text, the method can specifically include: inputting the multi-turn dialogue text that is entered by the user and that includes the target dialogue text and a predetermined slot filling instruction into the LLM model, so that the LLM model extracts, from the multi-turn dialogue text based on the slot filling instruction, the slot values of the plurality of predetermined slots corresponding to the target entity, and outputs a plurality of triplets corresponding to the slot values of the plurality of predetermined slots. Each of the triplets can include the target entity, any predetermined slot, and a slot value of the predetermined slot, and can be denoted as (entity, slot, and slot value).

For example, a formula corresponding to the above-mentioned slot filling can be shown as follows:

$$SV = \{(c, s, v) \, | \, c \in C\} = LLM\,(I_{SV},\, p_{m-k:\,m+k},\, d_{m-k:\,m+k})$$

SV is a triplet set constructed by a plurality of obtained triplets, c is any target entity, C is an entity set including the target entity, (c, s, v) is any triplet, s represents a predetermined slot, v represents a slot value of the slot, $I_{SV}$ is a slot filling instruction, $p_{m-k:\,m+k}$ is dialogue text entered by the user from an $(m-k)^{th}$ turn to an $(m+k)^{th}$ turn (i.e., the dialogue text from 2k turns before and after the target dialogue text in the $m^{th}$ turn), $d_{m-k:\,m+k}$ is an $(m-k)^{th}$ turn to an $(m+k)^{th}$ turn of dialogue text outputted by the LLM model, where k can be an integer greater than or equal to 1.

It should be understood that the attribute values of the plurality of attributes of the target entity may be mentioned in current target dialogue text and previous historical dialogue text of the user, but it is also very likely that the user provides related descriptions in a subsequent dialogue. Therefore, for a target entity in the current target dialogue text, a slot filling range of the target entity needs to be extended to a plurality of turns of dialogues that are before and after a current dialogue. In this way, even if the attribute values (namely, the slot values) of the plurality of attributes of the target entity cannot be obtained temporarily in the current target dialogue text and the historical dialogue text, slot filling can still be performed for the target entity in a subsequent plurality of turns of dialogues to obtain the slot values, so that sufficient fine-grained information is obtained as much as possible, without omitting any details provided by the user.

In addition, it is worthwhile to note that specific content of the slot filling instruction $I_{SV}$ is not particularly limited in this specification.

In a shown implementation, the slot filling instruction $I_{SV}$ can include a slot filling prompt and a plurality of examples. For example, the slot filling instruction $I_{SV}$ can be shown as follows:

You are an experienced doctor. Based on the context of the medical dialogue, identify a status of the given disease/symptom and return a result in a JSON format.

Main status: Candidate types come from ["Patient-reported positive", "Patient-reported negative", "Doctor-diagnosed positive", "Doctor-diagnosed negative", "Unknown"]

[Description of the main status types]

Medical history: Candidate types come from ["Yes", "No"]

[Description of medical history types]

Other relevant information: Other information, such as duration and a body part, that is mentioned for the given disease/symptom in the dialogue. Store the information in a list format.

Step S303: Construct, based on the subgraph and the attribute values of the plurality of attributes of the target entity, a dialogue graph corresponding to the target dialogue text.

Further, the dialogue graph corresponding to the target dialogue text can be constructed based on the obtained subgraph that is related to the target entity and that is in the knowledge graph and the attribute values (for example, the above-mentioned plurality of triplets) of the plurality of attributes of the target entity. In this way, the constructed dialogue graph not only includes specialized knowledge in the knowledge graph, but also includes fine-grained information provided by the user in the plurality of turns of dialogues.

In a shown implementation, the target entity in the dialogue graph can be connected to the plurality of neighboring nodes included in the subgraph (in other words, shared for the neighboring nodes in the knowledge graph), and the target entity is connected to the plurality of slot values included in the above-mentioned plurality of triplets by using an edge whose edge type is a corresponding predetermined slot. In a shown implementation, the dialogue graph further includes a user node, and the user node is connected to the target entity by using an edge whose edge type is an entity type of the target entity.

It should be understood that, because the user usually does not express too much information in the first turn of dialogue, based on the first turn of dialogue text of the user, an initial empty graph can be constructed first, and a user node can be created in the empty graph. With the plurality of turns of dialogues gradually developed between the user and the LLM model, the computing device can obtain more entities and more abundant information from the dialogue, thereby gradually expanding and enriching content in the dialogue graph.

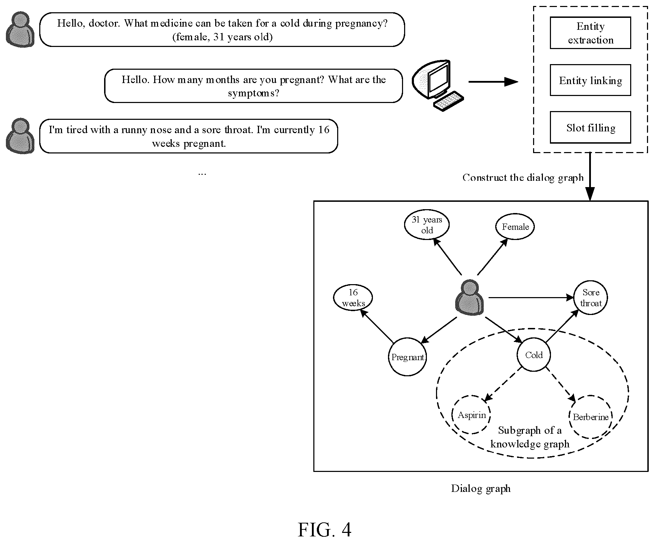

Referring to FIG. 4, FIG. 4 is a schematic flowchart illustrating construction of a dialogue graph, according to some example embodiments. As shown in FIG. 4, based on a plurality of turns of dialogues between a user and an LLM model, a constructed dialogue graph can include a user node located in a center and a plurality of nodes connected to the user node. The above-mentioned target entity can be "Pregnant", "Cold", and "Sore throat" shown in FIG. 4, and "Pregnant" duration is 16 weeks. Neighboring nodes in the subgraph can be "Aspirin" and "Berberine" shown in FIG. 4.

As shown in FIG. 4, an edge type of an edge connected between the user node and "Cold" can be "Disease", which is an entity type of the entity "Cold". An edge type of an edge connected between "Pregnant" and "16 weeks" can be "Duration", which is a predetermined slot corresponding to the entity "Pregnant". In addition, an edge type of an edge connected between "Cold" and "Aspirin" can be "Treatment method", an edge type of an edge connected between "Cold" and "Berberine" can be "Treatment method", etc. Details are not described here again.

In addition, in some possible implementations, in addition to performing entity extraction on dialogue text entered by the user, entity extraction and subsequent entity linking and slot filling can be further performed on dialogue text output by the LLM model in the plurality of turns of dialogues, so that information in the dialogue graph can be further enriched. This is not specifically limited in this specification.

Step S304: Construct a prompt based on the dialogue graph and the target dialogue text, and input the prompt into the LLM model, so that the LLM model performs logical inference based on the prompt.

Further, after the dialogue graph is obtained through construction, the prompt can be constructed based on the dialogue graph and the target dialogue text entered by the user, and the prompt is inputted into the LLM model, so that the LLM model performs logical inference based on the prompt, and outputs a corresponding inference result, in other words, outputs dialogue text that responds to the target dialogue text.

For example, an example in which a target application scenario is a medical consultation scenario is used. Logical inference performed by the LLM model can include logical inference related to a multi-turn dialogue task corresponding to the medical consultation scenario, such as symptom analysis, disease diagnosis, treatment advice, and medicine recommendation. This is not specifically limited in this specification.

In a shown implementation, after obtaining the dialogue graph through construction, the computing device can further generate, based on the dialogue graph, prompt information related to the target dialogue text. Correspondingly, the prompt can be constructed based on the dialogue graph, the prompt information, and the target dialogue text together, and the prompt is inputted into the LLM model, so that the LLM model performs logical inference based on the prompt. In this way, key information obtained through mining from the dialogue graph is further combined as a prompt, so that the LLM model can be more comprehensively and efficiently assisted to perform logical inference, thereby further improving inference performance of the model.

With reference to a specific example, the following describes a solution for generating the prompt information based on the dialogue graph provided in this application.

First, it is worthwhile to note that a specific implementation of generating the prompt information based on the dialogue graph is not particularly limited in this specification.

In a shown implementation, the prompt information can include prompt text used to describe a logical relationship between the target entity and at least part of neighboring nodes in the plurality of neighboring nodes.

Correspondingly, when the prompt information is generated based on the dialogue graph, the method can specifically include: determining an edge type of an edge connected between the target entity and the plurality of neighboring nodes (for example, first-order neighboring nodes) included in the dialogue graph, selecting, from the plurality of neighboring nodes, at least part of neighboring nodes whose edge types match a user intention, and then generating the prompt text used to describe the logical relationship between the target entity and the at least part of neighboring nodes.

In a shown implementation, when the at least part of neighboring nodes whose edge types match the user intention are selected from the plurality of neighboring nodes, the method can specifically include: inputting edge types (for example, a treatment method, a related symptom, and a precaution) of edges corresponding to the plurality of neighboring nodes into the LLM model, and prompting the LLM model to perform selection. If the LLM model selects a target type (for example, the "treatment method") from a plurality of edge types based on a user intention obtained through inference, at least part of neighboring nodes connected to the target entity by using edges of the target type can be obtained through filtering from the plurality of neighboring nodes. Further, corresponding prompt text used to describe a logical relationship between the target entity and the at least part of neighboring nodes can be generated based on a plurality of triplets including the target entity, the edges of the target type, and the at least part of neighboring nodes.

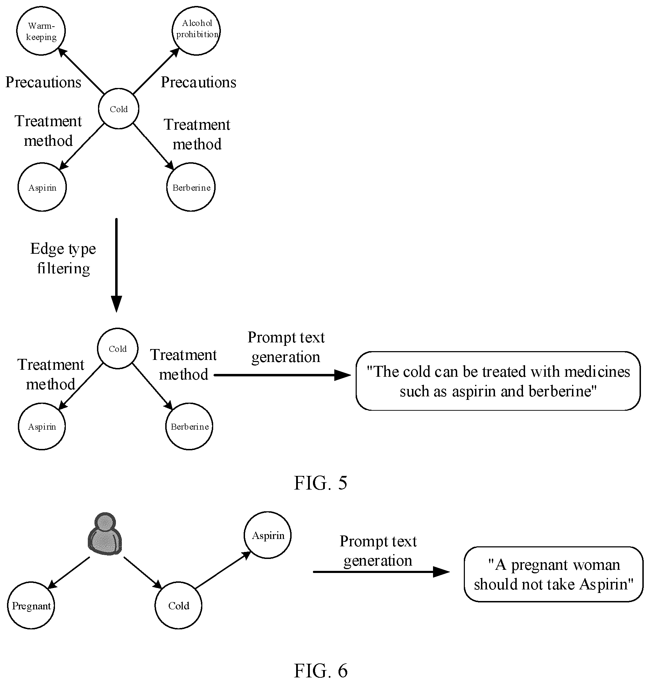

For example, referring to FIG. 5, FIG. 5 is a diagram illustrating generation of prompt text based on a dialogue graph, according to some example embodiments. FIG. 5 still uses the dialogue graph shown in FIG. 4 as an example, where a target entity included in the dialogue graph can be "Cold", and a plurality of neighboring nodes in a subgraph connected to "Cold" can include "Aspirin", "Berberine", "Warm-keeping", and "Alcohol prohibition". An edge type of an edge connected between the neighboring nodes "Aspirin" and "Berberine" and the target entity "Cold" is "Treatment method". An edge type of an edge connected between the neighboring nodes "Warm-keeping" and "Alcohol prohibition" and the target entity "Cold" is "Precautions". Based on this, the two edge types: "Treatment method" and "Precautions", can be inputted into an LLM model and the LLM model is prompted to perform selection. As shown in FIG. 5, if the LLM model selects "Treatment method", corresponding prompt text can be generated based on triplets (Cold, Treatment method, Aspirin) and (Cold, Treatment method, Berberine), for example, "The cold can be treated with medicines such as aspirin and berberine".

In a shown implementation, the prompt information can further include prompt text used to describe a conflict existed between information included in the dialogue graph and other information. The other information can be specialized knowledge information related to a target application scenario, known historical experience information, etc. This is not specifically limited in this specification.

Correspondingly, when the prompt information is generated based on the dialogue graph, the method can specifically include: obtaining a pre-built prompt path, where the prompt path can include a plurality of nodes connected in sequence; further, determining whether the dialogue graph includes a path that matches the prompt path; if included, querying a knowledge graph for information related to the plurality of nodes included in the path; and further, in response to a conflict existed between identified information in the knowledge graph and information included in the path, generating prompt text used to describe the conflict.

For example, the prompt path can be, for example, Pregnant ← User → Hypertension → Medicine or Pregnant ← User → Cold → Medicine. This is not specifically limited in this specification.

For example, referring to FIG. 6, FIG. 6 is another diagram illustrating generation of prompt text based on a dialogue graph, according to some example embodiments. FIG. 6 still uses the dialogue graph shown in FIG. 4 as an example. Assume that a predetermined prompt path is: Pregnant ← User → Cold → Medicine. As shown in FIG. 6, a path (Pregnant ← User → Cold → Aspirin) that matches the prompt path is found in the dialogue graph. Further, a knowledge graph can be queried for information related to a plurality of nodes included in the path, for example, queried for a medicine use description of aspirin, and precautions of a cold during pregnancy. For example, if information identified from the knowledge graph can include that a pregnant woman should not take aspirin, prompt text, for example, "A pregnant woman should not take aspirin", used to describe a conflict between the information in the knowledge graph and information included in the path can be generated based on the conflict.

In a shown implementation, a conflict existed between information included in the dialogue graph and other information may be difficult to simply obtain by querying the knowledge graph, or a conflict existed between information included in the dialogue graph and other information cannot be directly discovered when inference about more fine-grained medical information is involved.

Based on this, after it is also determined that the dialogue graph includes the path that matches the predetermined prompt path, question text corresponding to the path can be first generated based on the information included in the path, in other words, the matched path is converted into a natural language query.

Further, the question text that is generated can be inputted into an LLM model, so that the LLM model infers, based on the question text, a conflict that may be existed between the information included in the path and other information, and outputs prompt text used to describe the conflict. In this way, the LLM model can be encouraged to perform additional thinking to try to infer the conflict existed between the information included in the path and other information.

Alternatively, the question text that is generated can be inputted into an Internet search engine, to identify, by using the search engine, a conflict existed between the information included in the path and other information, and the prompt text used to describe the conflict is generated based on a query result.

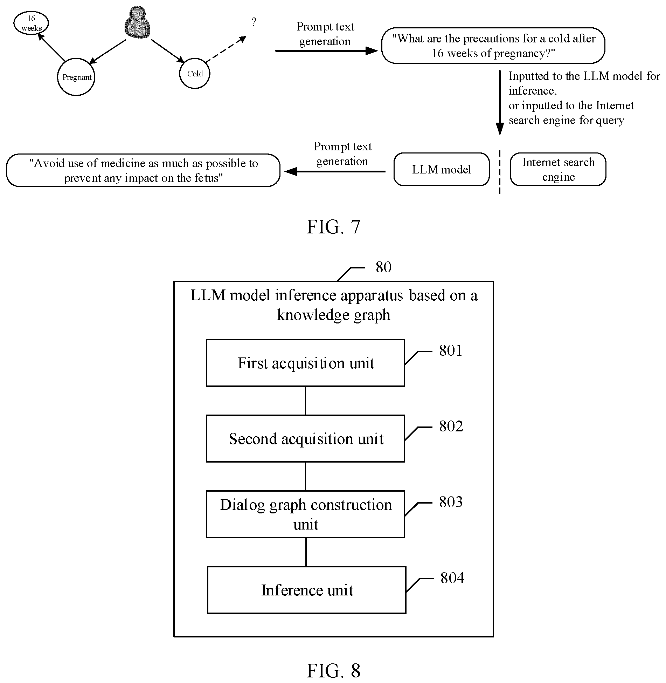

For example, referring to FIG. 7, FIG. 7 is still another diagram illustrating generation of prompt text based on a dialogue graph, according to some example embodiments. FIG. 7 still uses the dialogue graph shown in FIG. 4 as an example. Assume that a predetermined prompt path is: Duration ← Pregnant ← User → Cold. As shown in FIG. 7, a path (16 weeks ← Pregnant ← User → Cold) that matches the prompt path is identified in the dialogue graph. Further, as shown in FIG. 7, question text corresponding to the path can be generated based on information included in the path, for example, "What are the precautions for a cold after 16 weeks of pregnancy". Further, the question text can be inputted into an LLM model for inference or inputted into an Internet search engine for query, to obtain prompt text, such as " Try to avoid using medication to prevent any impact on the fetus", used to describe a conflict existed between information included in the path and other information. This is not specifically limited in this specification.

In a shown implementation, in this application, a plurality of prompt paths can be predefined, and path matching and prompt text generation are concurrently performed based on the plurality of prompt paths, etc., to improve generation efficiency of the prompt text, thereby enriching a dialogue policy of the LLM model, etc. This is not specifically limited in this specification.

In conclusion, in this application, the dialogue graph is constructed by using a pipeline framework, which not only explicitly captures fine-grained information in a dialogue stream (to be specific, a plurality of turns of dialogues), preventing forgetfulness of a large model, but also masters specialized knowledge in a knowledge graph. The dialogue graph can not only be used for retrieval-augmented generation (RAG), generation of a medical record in an electronic form, etc., but also be adapted to a medical dialogue system framework in a large model era, which can provide a better medical service for a patient. In addition, the method provided in this application merely prompts the large model by using knowledge graph-based RAG and context learning, and does not need to be adapted to another hardware or software method. This is "Plug-and-play". Deployment costs are small, and an application scope is wide. In addition to being applied to the above-mentioned medical consultation scenario, the method can be further applied to various scenarios such as life assistant and emotion analysis, to provide a user with a high quality of service.

Corresponding to the above-mentioned method procedure implementation, some embodiments of this specification further provide an LLM model inference apparatus based on a knowledge graph. The apparatus can be applied to the computing device shown in FIG. 2. Referring to FIG. 8, FIG. 8 is a diagram illustrating a structure of an LLM model inference apparatus based on a knowledge graph, according to some example embodiments. As shown in FIG. 8, the apparatus 80 includes: a first acquisition unit 801, configured to obtain target dialogue text entered by a user, and identify, from the knowledge graph, a subgraph related to an entity mention included in the target dialogue text, where the subgraph includes a target node that represents a target entity and a plurality of neighboring nodes connected to the target node; a second acquisition unit 802, configured to obtain attribute values of a plurality of attributes of the target entity from multi-turn dialogue text that is entered by the user and that includes the target dialogue text; a dialogue graph construction unit 803, configured to construct, based on the subgraph and the attribute values of the plurality of attributes of the target entity, a dialogue graph corresponding to the target dialogue text; and an inference unit 804, configured to construct a prompt based on the dialogue graph and the target dialogue text, and input the prompt into an LLM model, so that the LLM model performs logical inference based on the prompt.

In a shown implementation, the first acquisition unit 801 is specifically configured to: input the target dialogue text and a predetermined entity extraction instruction into the LLM model, so that the LLM model extracts, based on the entity extraction instruction, the included entity mention from the target dialogue text; and determine, through entity linking, a target entity that is included in the knowledge graph and that corresponds to the entity mention, and identify a subgraph related to the target entity from the knowledge graph.

In a shown implementation, the second acquisition unit 802 is specifically configured to: determine the plurality of attributes of the target entity based on an entity type of the target entity; and obtain a plurality of predetermined slots constructed for the plurality of attributes of the target entity, and obtain, through slot filling, slot values of the plurality of predetermined slots corresponding to the target entity from the multi-turn dialogue text that is entered by the user and that includes the target dialogue text.

In a shown implementation, the second acquisition unit 802 is specifically configured to: input the multi-turn dialogue text that is entered by the user and that includes the target dialogue text and a predetermined slot filling instruction into the LLM model, so that the LLM model extracts, from the multi-turn dialogue text based on the slot filling instruction, the slot values of the plurality of predetermined slots corresponding to the target entity, and outputs a plurality of triplets corresponding to the slot values of the plurality of predetermined slots, where each of the triplets includes the target entity, a predetermined slot, and a slot value of the predetermined slot.

In a shown implementation, the dialogue graph construction unit 803 is specifically configured to construct, based on the subgraph and the plurality of triplets, the dialogue graph corresponding to the target dialogue text, where the target entity in the dialogue graph is connected to the plurality of neighboring nodes included in the subgraph, and the target entity is connected, by using an edge whose edge type is a corresponding predetermined slot, to the plurality of slot values included in the plurality of triplets; and the dialogue graph further includes a user node, and the user node is connected to the target entity by using an edge whose edge type is an entity type of the target entity.

In a shown implementation, the inference unit 804 is specifically configured to: generate, based on the dialogue graph, prompt information related to the target dialogue text; and construct the prompt based on the dialogue graph, the prompt information, and the target dialogue text, and input the prompt into the LLM model.

In a shown implementation, the prompt information includes prompt text used to describe a logical relationship between the target entity and at least part of neighboring nodes in the plurality of neighboring nodes; and the inference unit 804 is specifically configured to: determine an edge type of an edge that is included in the dialogue graph and that is connected between the target entity and the plurality of neighboring nodes; and select, from the plurality of neighboring nodes, at least part of neighboring nodes whose edge types match a user intention, and generate the prompt text used to describe the logical relationship between the target entity and the at least part of neighboring nodes.

In a shown implementation, the prompt information includes prompt text used to describe a conflict existed between information included in the dialogue graph and other information; and the inference unit 804 is specifically configured to: obtain a pre-built prompt path, where the prompt path includes a plurality of nodes connected in sequence; determine whether the dialogue graph includes a path that matches the prompt path; and if included, query the knowledge graph for information related to a plurality of nodes included in the path; and generate, in response to a conflict existed between identified information in the knowledge graph and information included in the path, the prompt text used to describe the conflict.

In a shown implementation, the prompt information includes prompt text used to describe a conflict existed between information included in the dialogue graph and other information; and the inference unit 804 is specifically configured to: obtain a pre-built prompt path, where the prompt path includes a plurality of nodes connected in sequence; determine whether the dialogue graph includes a path that matches the prompt path; and if included, generate, based on information included in the path, question text corresponding to the path; and input the question text into the LLM model, so that the LLM model infers, based on the question text, a conflict existed between the information included in the path and other information, and outputs the prompt text used to describe the conflict; or input the question text into an Internet search engine, to identify a conflict existed between the information included in the path and other information, and generate, based on a query result, the prompt text used to describe the conflict.

In a shown implementation, the LLM model is an LLM service model obtained after a pre-trained LLM base model is fine-tuned based on data related to a target application scenario.

In a shown implementation, the target application scenario includes a medical consultation scenario, the logical inference includes logical inference related to a multi-turn dialogue task corresponding to the medical consultation scenario, and the knowledge graph is a knowledge graph in a medical field.

For a specific implementation process of functions and effects of units in the apparatus 80, refer to the descriptions of the above-mentioned embodiments. Details are not described here again. It should be understood that the apparatus 80 can be implemented by using software, or can be implemented by using hardware or a combination of software and hardware. Software implementation is used as an example. As a logical apparatus, the apparatus is formed by reading corresponding computer program instructions to a memory by a processor (CPU) of a device in which the apparatus is located. In terms of hardware, in addition to a CPU and a storage, the device in which the apparatus is located usually further includes other hardware such as a chip for sending and receiving radio signals, and/or other hardware such as a card for implementing a network communication function.

The described apparatus embodiment is merely an example. The units described as separate parts can or do not have to be physically separate, and parts displayed as units can or do not have to be physical modules, can be located in one position, or can be distributed on a plurality of network modules. Some or all of the units or modules can be selected based on actual needs to implement the objectives of the solutions of this specification. A person of ordinary skill in the art can understand and implement the embodiments without creative efforts.

The apparatuses, units, or modules described in the above-mentioned embodiments can be implemented by a computer chip or an entity, or can be implemented by a product with a certain function. A typical implementation device is a computer, and a specific form of the computer can be a personal computer, a laptop computer, a cellular phone, a camera phone, a smartphone, a personal digital assistant, a media player, a navigation device, an email sending and receiving device, a game console, a tablet computer, a wearable device, or any combination of a plurality of these devices.

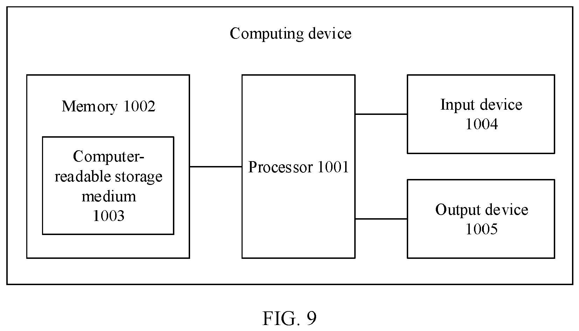

Corresponding to the above-mentioned method embodiments, some embodiments of this specification further provide a computing device. Referring to FIG. 9, FIG. 9 is a diagram illustrating a structure of a computing device, according to some example embodiments. As shown in FIG. 9, the computing device includes a processor 1001 and a memory 1002, and can further include an input device 1004 (such as a keyboard) and an output device 1005 (such as a display). The processor 1001, the memory 1002, the input device 1004, and the output device 1005 can be connected by using a bus or in another way. As shown in FIG. 9, the memory 1002 includes a computer-readable storage medium 1003, and the computer-readable storage medium 1003 stores a computer program executable by the processor 1001. The processor 1001 can be a CPU, a microprocessor, or an integrated circuit for controlling the execution of the above-mentioned method embodiments. When running a stored computer program, the processor 1001 can perform steps of the LLM model inference method based on a knowledge graph in embodiments of this specification, including: obtaining target dialogue text entered by a user, and identifying, from the knowledge graph, a subgraph related to an entity mention included in the target dialogue text, where the subgraph includes a target node that represents a target entity and a plurality of neighboring nodes connected to the target node; obtaining attribute values of a plurality of attributes of the target entity from multi-turn dialogue text that is entered by the user and that includes the target dialogue text; constructing, based on the subgraph and the attribute values of the plurality of attributes of the target entity, a dialogue graph corresponding to the target dialogue text; and constructing a prompt based on the dialogue graph and the target dialogue text, and inputting the prompt into an LLM model, so that the LLM model performs logical inference based on the prompt, etc.

For detailed descriptions of the steps of the above-mentioned LLM model inference method based on a knowledge graph, refer to the above-mentioned content. Details are not described here again.

Corresponding to the above-mentioned method embodiments, some embodiments of this specification further provide a computer-readable storage medium. The storage medium stores a computer program. When the computer program is run by a processor, steps of the LLM model inference method based on a knowledge graph in embodiments of this specification are performed. For details, refer to the descriptions of the above-mentioned embodiments. Details are not described here again.

The above-mentioned descriptions are merely preferred embodiments of this specification, but are not intended to limit this specification. Any modification, equivalent replacement, or improvement made within the spirit and principles of this specification shall be included in the protection scope of this specification.

In a typical configuration, the computing device includes one or more CPUs, input/output interfaces, network interfaces, and memories.

The memory may include a form such as a non-persistent memory, a random access memory (RAM), and/or a non-volatile memory in computer-readable media, such as a read-only memory (ROM) or a flash memory (flash RAM). The memory is an example of the computer-readable media.

The computer-readable media includes persistent and non-persistent, removable, and non-removable media, can be implemented by any method or technology for information storage. The information can be a computer-readable instruction, a data structure, a program module, or other data.

Examples of a storage medium of a computer include but are not limited to a phase-change random access memory (PRAM), a static random access memory (SRAM), a dynamic random access memory (DRAM), another type of random access memory (RAM), a read-only memory (ROM), an electrically erasable programmable read-only memory (EEPROM), a flash memory or another memory technology, a compact disc read-only memory (CD-ROM), a digital versatile disk (DVD) or another optical storage, a magnetic cassette tape, a magnetic disk storage or another magnetic storage device, or any other non-transmission medium, which can be configured to store information accessible to a computing device. As described in this specification, the computer-readable medium does not include computer-readable transitory media such as a modulated data signal and a carrier.

It is worthwhile to further note that the terms "include", "contain" or their any other variants are intended to cover non-exclusive inclusion, so that a process, a method, a product, or a device that includes a list of elements not only includes those elements but also includes other elements that are not explicitly listed, or further includes elements inherent to such a process, method, product, or device. Without more constraints, an element preceded by "includes a ... " does not preclude the existence of additional identical elements in the process, method, product, or device that includes the element.

A person skilled in the art should understand that the embodiments of this specification can be provided as a method, a system, or a computer program product. Therefore, the embodiments of this specification can use a form of hardware only embodiments, software only embodiments, or embodiments with a combination of software and hardware. Moreover, the embodiments of this specification can use a form of a computer program product that is implemented on one or more computer-usable storage media (including but not limited to a disk memory, a CD-ROM, an optical memory, etc.) that include computer-usable program code.

**EXAMPLES**

1. An LLM model inference method based on a knowledge graph, wherein the knowledge graph comprises a plurality of nodes and edges connecting the nodes, the nodes represent entities, and the edges represent relationships between the entities; and the method comprises:

> obtaining target dialogue text entered by a user, and identifying, from the knowledge graph, a subgraph related to an entity mention comprised in the target dialogue text, wherein the subgraph comprises a target node that represents a target entity and a plurality of neighboring nodes connected to the target node;
> obtaining attribute values of a plurality of attributes of the target entity from multi-turn dialogue text that is entered by the user and that comprises the target dialogue text;
> constructing, based on the subgraph and the attribute values of the plurality of attributes of the target entity, a dialogue graph corresponding to the target dialogue text; and
> constructing a prompt based on the dialogue graph and the target dialogue text, and inputting the prompt into an LLM model, so that the LLM model performs logical inference based on the prompt.

2. The method according to example 1, wherein the identifying, from the knowledge graph, a subgraph related to an entity mention comprised in the target dialogue text comprises:

> inputting the target dialogue text and a predetermined entity extraction instruction into the LLM model, so that the LLM model extracts, based on the entity extraction instruction, the comprised entity mention from the target dialogue text; and
> determining, through entity linking, a target entity that is comprised in the knowledge graph and that corresponds to the entity mention, and identifying a subgraph related to the target entity from the knowledge graph.

3. The method according to example 1, wherein the obtaining attribute values of a plurality of attributes of the target entity from multi-turn dialogue text that is entered by the user and that comprises the target dialogue text comprises:

> determining the plurality of attributes of the target entity based on an entity type of the target entity; and
> obtaining a plurality of predetermined slots constructed for the plurality of attributes of the target entity, and obtaining, through slot filling, slot values of the plurality of predetermined slots corresponding to the target entity from the multi-turn dialogue text that is entered by the user and that comprises the target dialogue text.

4. The method according to example 3, wherein the obtaining, through slot filling, slot values of the plurality of predetermined slots corresponding to the target entity from the multi-turn dialogue text that is entered by the user and that comprises the target dialogue text comprises:

inputting the multi-turn dialogue text that is entered by the user and that comprises the target dialogue text and a predetermined slot filling instruction into the LLM model, so that the LLM model extracts, from the multi-turn dialogue text based on the slot filling instruction, the slot values of the plurality of predetermined slots corresponding to the target entity, and outputs a plurality of triplets corresponding to the slot values of the plurality of predetermined slots, wherein

each of the triplets comprises the target entity, a predetermined slot, and a slot value of the predetermined slot.

5. The method according to example 4, wherein the constructing, based on the subgraph and the attribute values of the plurality of attributes of the target entity, a dialogue graph corresponding to the target dialogue text comprises: constructing, based on the subgraph and the plurality of triplets, the dialogue graph corresponding to the target dialogue text, wherein the target entity in the dialogue graph is connected to the plurality of neighboring nodes comprised in the subgraph, and the target entity is connected, by using an edge whose edge type is a corresponding predetermined slot, to the plurality of slot values comprised in the plurality of triplets; and the dialogue graph further comprises a user node, and the user node is connected to the target entity by using an edge whose edge type is an entity type of the target entity.

6. The method according to example 1, wherein the constructing a prompt based on the dialogue graph and the target dialogue text, and inputting the prompt into an LLM model comprises:

generating, based on the dialogue graph, prompt information related to the target dialogue text; and constructing the prompt based on the dialogue graph, the prompt information, and the target dialogue text, and inputting the prompt into the LLM model.

7. The method according to example 6, wherein the prompt information comprises prompt text used to describe a logical relationship between the target entity and at least part of neighboring nodes in the plurality of neighboring nodes; and the generating, based on the dialogue graph, prompt information related to the target dialogue text comprises:

determining an edge type of an edge that is comprised in the dialogue graph and that is connected between the target entity and the plurality of neighboring nodes; and

selecting, from the plurality of neighboring nodes, at least part of neighboring nodes whose edge types match a user intention, and generating the prompt text used to describe the logical relationship between the target entity and the at least part of neighboring nodes.

8. The method according to example 6, wherein the prompt information comprises prompt text used to describe a conflict existed between information comprised in the dialogue graph and other information; and the generating, based on the dialogue graph, prompt information related to the target dialogue text comprises:

obtaining a pre-built prompt path, wherein the prompt path comprises a plurality of nodes connected in sequence; determining whether the dialogue graph comprises a path that matches the prompt path; and if comprised, querying the knowledge graph for information related to a plurality of nodes comprised in the path; and generating, in response to a conflict existed between identified information in the knowledge graph and information comprised in the path, the prompt text used to describe the conflict.

9. The method according to example 6, wherein the prompt information comprises prompt text used to describe a conflict existed between information comprised in the dialogue graph and other information; and the generating, based on the dialogue graph, prompt information related to the target dialogue text comprises:

obtaining a pre-built prompt path, wherein the prompt path comprises a plurality of nodes connected in sequence; determining whether the dialogue graph comprises a path that matches the prompt path; and if comprised, generating, based on information comprised in the path, question text corresponding to the path; and inputting the question text into the LLM model, so that the LLM model infers, based on the question text, a conflict existed between the information comprised in the path and other information, and outputs the prompt text used to describe the conflict; or

inputting the question text into an Internet search engine, to identify a conflict existed between the information comprised in the path and other information, and generating, based on a query result, the prompt text used to describe the conflict.

10. The method according to any one of examples 1 to 9, wherein the LLM model is an LLM service model obtained after a pre-trained LLM base model is fine-tuned based on data related to a target application scenario.

11. The method according to example 10, wherein the target application scenario comprises a medical consultation scenario, the logical reference comprises logical reference related to a multi-turn dialogue task corresponding to the medical consultation scenario, and the knowledge graph is a knowledge graph in a medical field.

12. An LLM model inference apparatus based on a knowledge graph, wherein the knowledge graph comprises a plurality of nodes and edges connecting the nodes, the nodes represent entities, and the edges represent relationships between the entities; and the apparatus comprises:

a first acquisition unit, configured to obtain target dialogue text entered by a user, and identify, from the knowledge graph, a subgraph related to an entity mention comprised in the target dialogue text, wherein the subgraph comprises a target node that represents a target entity and a plurality of neighboring nodes connected to the target node;
a second acquisition unit, configured to obtain attribute values of a plurality of attributes of the target entity from multi-turn dialogue text that is entered by the user and that comprises the target dialogue text;
a dialogue graph construction unit, configured to construct, based on the subgraph and the attribute values of the plurality of attributes of the target entity, a dialogue graph corresponding to the target dialogue text; and
an inference unit, configured to construct a prompt based on the dialogue graph and the target dialogue text, and input the prompt into an LLM model, so that the LLM model performs logical inference based on the prompt.

13. A computing device, comprising a memory and a processor, wherein the memory stores a computer program/instructions that can be run by the processor; and when running the computer program/instructions, the processor executes the method according to any one of examples 1 to 11.

14. A computer-readable storage medium, wherein a computer program/instructions is/are stored on the computer-readable storage medium, and when being executed by a processor, the computer program/instructions implements/implement the method according to any one of examples 1 to 11.

15. A computer program product, wherein the computer program product comprises a computer program/instructions, and when being executed by a processor, the computer program/instructions implements/implement the method according to any one of examples 1 to 11.

## Claims

1. An LLM model inference method based on a knowledge graph, wherein the knowledge graph comprises a plurality of nodes and edges connecting the nodes, the nodes represent entities, and the edges represent relationships between the entities; and the method comprises:

obtaining target dialogue text entered by a user, and identifying, from the knowledge graph, a subgraph related to an entity mention comprised in the target dialogue text, wherein the subgraph comprises a target node that represents a target entity and a plurality of neighboring nodes connected to the target node;
obtaining attribute values of a plurality of attributes of the target entity from multi-turn dialogue text that is entered by the user and that comprises the target dialogue text;
constructing, based on the subgraph and the attribute values of the plurality of attributes of the target entity, a dialogue graph corresponding to the target dialogue text; and
constructing a prompt based on the dialogue graph and the target dialogue text, and inputting the prompt into an LLM model, so that the LLM model performs logical inference based on the prompt.

2. The method according to claim 1, wherein identifying, from the knowledge graph, a subgraph related to an entity mention comprised in the target dialogue text comprises:

inputting the target dialogue text and a predetermined entity extraction instruction into the LLM model, so that the LLM model extracts, based on the entity extraction instruction, the comprised entity mention from the target dialogue text; and
determining, through entity linking, a target entity that is comprised in the knowledge graph and that corresponds to the entity mention, and identifying a subgraph related to the target entity from the knowledge graph.

3. The method according to claim 1 or 2, wherein obtaining attribute values of a plurality of attributes of the target entity from multi-turn dialogue text that is entered by the user and that comprises the target dialogue text comprises:

   determining the plurality of attributes of the target entity based on an entity type of the target entity; and obtaining a plurality of predetermined slots constructed for the plurality of attributes of the target entity, and obtaining, through slot filling, slot values of the plurality of predetermined slots corresponding to the target entity from the multi-turn dialogue text that is entered by the user and that comprises the target dialogue text.

4. The method according to claim 3, wherein obtaining, through slot filling, slot values of the plurality of predetermined slots corresponding to the target entity from the multi-turn dialogue text that is entered by the user and that comprises the target dialogue text comprises:

   inputting the multi-turn dialogue text that is entered by the user and that comprises the target dialogue text and a predetermined slot filling instruction into the LLM model, so that the LLM model extracts, from the multi-turn dialogue text based on the slot filling instruction, the slot values of the plurality of predetermined slots corresponding to the target entity, and outputs a plurality of triplets corresponding to the slot values of the plurality of predetermined slots, wherein
   each of the triplets comprises the target entity, a predetermined slot, and a slot value of the predetermined slot.

5. The method according to claim 4, wherein constructing, based on the subgraph and the attribute values of the plurality of attributes of the target entity, a dialogue graph corresponding to the target dialogue text comprises:
   constructing, based on the subgraph and the plurality of triplets, the dialogue graph corresponding to the target dialogue text, wherein the target entity in the dialogue graph is connected to the plurality of neighboring nodes comprised in the subgraph, and the target entity is connected, by using an edge whose edge type is a corresponding predetermined slot, to the plurality of slot values comprised in the plurality of triplets; and the dialogue graph further comprises a user node, and the user node is connected to the target entity by using an edge whose edge type is an entity type of the target entity.

6. The method according to any one of claims 1 to 5, wherein constructing a prompt based on the dialogue graph and the target dialogue text, and inputting the prompt into an LLM model comprises:

   generating, based on the dialogue graph, prompt information related to the target dialogue text; and constructing the prompt based on the dialogue graph, the prompt information, and the target dialogue text, and inputting the prompt into the LLM model.

7. The method according to claim 6, wherein the prompt information comprises prompt text used to describe a logical relationship between the target entity and at least part of neighboring nodes in the plurality of neighboring nodes; and the generating, based on the dialogue graph, prompt information related to the target dialogue text comprises:

   determining an edge type of an edge that is comprised in the dialogue graph and that is connected between the target entity and the plurality of neighboring nodes; and
   selecting, from the plurality of neighboring nodes, at least part of neighboring nodes whose edge types match a user intention, and generating the prompt text used to describe the logical relationship between the target entity and the at least part of neighboring nodes.

8. The method according to claim 6, wherein the prompt information comprises prompt text used to describe a conflict existed between information comprised in the dialogue graph and other information; and the generating, based on the dialogue graph, prompt information related to the target dialogue text comprises:

   obtaining a pre-built prompt path, wherein the prompt path comprises a plurality of nodes connected in sequence; determining whether the dialogue graph comprises a path that matches the prompt path; and if comprised, querying the knowledge graph for information related to a plurality of nodes comprised in the path; and generating, in response to a conflict existed between identified information in the knowledge graph and information comprised in the path, the prompt text used to describe the conflict.

9. The method according to claim 6, wherein the prompt information comprises prompt text used to describe a conflict existed between information comprised in the dialogue graph and other information; and the generating, based on the dialogue graph, prompt information related to the target dialogue text comprises:

> obtaining a pre-built prompt path, wherein the prompt path comprises a plurality of nodes connected in sequence; determining whether the dialogue graph comprises a path that matches the prompt path; and if comprised, generating, based on information comprised in the path, question text corresponding to the path; and inputting the question text into the LLM model, so that the LLM model infers, based on the question text, a conflict existed between the information comprised in the path and other information, and outputs the prompt text used to describe the conflict; or
> inputting the question text into an Internet search engine, to identify a conflict existed between the information comprised in the path and other information, and generating, based on a query result, the prompt text used to describe the conflict.

10. The method according to any one of claims 1 to 9, wherein the LLM model is an LLM service model obtained after a pre-trained LLM base model is fine-tuned based on data related to a target application scenario.

11. The method according to claim 10, wherein the target application scenario comprises a medical consultation scenario, the logical reference comprises logical reference related to a multi-turn dialogue task corresponding to the medical consultation scenario, and the knowledge graph is a knowledge graph in a medical field.

12. An LLM model inference apparatus based on a knowledge graph, wherein the knowledge graph comprises a plurality of nodes and edges connecting the nodes, the nodes represent entities, and the edges represent relationships between the entities; and the apparatus comprises:

> a first acquisition unit, configured to obtain target dialogue text entered by a user, and identify, from the knowledge graph, a subgraph related to an entity mention comprised in the target dialogue text, wherein the subgraph comprises a target node that represents a target entity and a plurality of neighboring nodes connected to the target node;
> a second acquisition unit, configured to obtain attribute values of a plurality of attributes of the target entity from multi-turn dialogue text that is entered by the user and that comprises the target dialogue text;
> a dialogue graph construction unit, configured to construct, based on the subgraph and the attribute values of the plurality of attributes of the target entity, a dialogue graph corresponding to the target dialogue text; and
> an inference unit, configured to construct a prompt based on the dialogue graph and the target dialogue text, and input the prompt into an LLM model, so that the LLM model performs logical inference based on the prompt.

13. A computing device, comprising a memory and a processor, wherein the memory stores a computer program/instructions that can be run by the processor; and when running the computer program/instructions, the processor executes the method according to any one of claims 1 to 11.

14. A computer-readable storage medium, wherein a computer program/instructions is/are stored on the computer-readable storage medium, and when being executed by a processor, the computer program/instructions implements/implement the method according to any one of claims 1 to 11.

15. A computer program product, wherein the computer program product comprises a computer program/instructions, and when being executed by a processor, the computer program/instructions implements/implement the method according to any one of claims 1 to 11.

My wife is pregnant for 30 weeks, and her blood pressure is now 145

How long has this situation been going on

Almost three days ago

Has she experienced any other discomfort before

She also has bronchitis. Does it have any impact

Labetalol can be considered for blood pressure control

Medication Recommendation

Labetalol is contraindicated or should be used with caution for a patient with non-allergic bronchitis.
Methyldopa is recommended for blood pressure control.

FIG. 1

Output an
answer
dialog to
the user

User
Input

Inputted to the
LLM model
for inference

Computing device
(An LLM model is mounted)

Target dialog
text

Dialog graph

Mining

Prompt
information

Entity
extraction

Entity
linking

Slot filling

Knowledge
graph

FIG. 2

Obtain target dialogue text entered by a user, and identify, from a knowledge graph, a subgraph related to an entity mention included in the target dialogue text, where the subgraph includes a target node that represents a target entity and a plurality of neighboring nodes connected to the target node

S301

Obtain attribute values of a plurality of attributes of the target entity from multi-turn dialogue text that is entered by the user and that includes the target dialogue text

S302

Construct, based on the subgraph and the attribute values of the plurality of attributes of the target entity, a dialogue graph corresponding to the target dialogue text

S303

Construct a prompt based on the dialogue graph and the target dialogue text, and input the prompt into an LLM model, so that the LLM model performs logical inference based on the prompt

S304

FIG. 3

Hello, doctor. What medicine can be taken for a cold during pregnancy?
(female, 31 years old)

Hello. How many months are you pregnant? What are the symptoms?

I'm tired with a runny nose and a sore throat. I'm currently 16 weeks pregnant.

...

Entity extraction

Entity linking

Slot filling

Construct the dialog graph

31 years old

Female

16 weeks

Sore throat

Pregnant

Cold

Aspirin

Berberine

Subgraph of a knowledge graph

Dialog graph

FIG. 4

FIG. 5

FIG. 6

FIG. 7

FIG. 8

Computing device

Memory 1002

Computer-
readable storage
medium
1003

Processor 1001

Input device
1004

Output device
1005

FIG. 9

**EUROPEAN SEARCH REPORT**

Application Number

EP 25 20 0782

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/311807 A1 (KANNAN ANITHA [US] ET AL) 10 October 2019 (2019-10-10) * Abstract, Title * * [0015],[0047]-[0048],[0054],[0074],[0076]-[0077],[0084],[0095],[0137] * * figures 1,7,15 * | 1-15 | INV. G06N5/022 G06N3/0475 |
| A | DI MARO MARIA ET AL: "Cutting melted butter? Common Ground inconsistencies management in dialogue systems using graph databases", ITALIAN JOURNAL OF COMPUTATIONAL LINGUISTICS, vol. 7, no. 1 ¦ 2, 1 December 2021 (2021-12-01), pages 157-190, XP093354774, ISSN: 2499-4553, DOI: 10.4000/ijcol.892 * Abstract * * p168 * | 8,9 | |
| A | KIM JINSUNG ET AL: "Prompt Language Learner with Trigger Generation for Dialogue Relation Extraction", APPLIED SCIENCES, vol. 13, no. 22, 16 November 2023 (2023-11-16), page 12414, XP093354856, Basel ISSN: 2076-3417, DOI: 10.3390/app132212414 * the whole document * | 1-15 | TECHNICAL FIELDS SEARCHED (IPC) G06N G16H |
| A | CN 118 095 449 B (ALIPAY HANGZHOU TECH CO LTD) 6 August 2024 (2024-08-06) * the whole document * | 1-15 | |
| A | CN 118 116 620 A (ALIPAY HANGZHOU TECH CO LTD) 31 May 2024 (2024-05-31) * the whole document * | 1-15 | |

The present search report has been drawn up for all claims

| Place of search | Date of completion of the search | Examiner |
|---|---|---|
| The Hague | 20 January 2026 | Veynachter, Alexis |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 20 0782

This annex lists the patent family members relating to the patent documents cited in the above-mentioned European search report.
The members are as contained in the European Patent Office EDP file on
The European Patent Office is in no way liable for these particulars which are merely given for the purpose of information.

20-01-2026

| Patent document cited in search report | | Publication date | Patent family member(s) | | Publication date |
|---|---|---|---|---|---|
| US 2019311807 A1 | | 10-10-2019 | US | 12169767 B1 | 17-12-2024 |
| | | | US | 2019311807 A1 | 10-10-2019 |
| | | | US | 2023105969 A1 | 06-04-2023 |
| | | | US | 2025132051 A1 | 24-04-2025 |
| | | | US | 2025157670 A1 | 15-05-2025 |
| CN 118095449 B | | 06-08-2024 | CN | 118095449 A | 28-05-2024 |
| | | | CN | 118674055 A | 20-09-2024 |
| | | | WO | 2025223523 A1 | 30-10-2025 |
| CN 118116620 A | | 31-05-2024 | NONE | | |

EPO FORM P0459

For more details about this annex : see Official Journal of the European Patent Office, No. 12/82